# EUROPEAN PATENT SPECIFICATION

(11) **EP 1 689 286 B1**
(45) Date of publication and mention of the grant of the patent: **09.01.2013**
(21) Application number: 04812587.6
(22) Date of filing: 01.12.2004
(51) Int. Cl.: A61B 5/055, G01R 33/381, G01R 33/30

(54) **STAND-UP VERTICAL FIELD MRI APPARATUS**
STEHENDES VERTIKALFELD-MRT-GERÄT
APPAREIL D'IRM DEBOUT A CHAMP VERTICAL

(30) Priority: 01.12.2003 US 526029 P
(43) Date of publication of application: 16.08.2006
(73) Proprietor: Fonar Corporation, Melville, NY 11747-4292 (US)
(72) Inventor: DAMADIAN, Raymond, V., Woodbury, NY 11797 (US)
(74) Representative: Schmidt, Karsten
(86) International application number: PCT/US2004/040104
(87) International publication number: WO 2005/053515

(56) References cited:
- EP-A- 0 067 933
- EP-A- 0 350 267
- WO-A-01/70109
- JP-A- 1 305 937
- JP-A- 2001 104 276
- US-A- 5 565 834
- US-A- 5 659 281
- US-A- 6 023 165
- US-B1- 6 288 546

## Description

### TECHNICAL FIELD

The present invention relates to magnetic resonance imaging apparatus.

In magnetic resonance imaging, an object to be imaged as, for example, a body of a human subject, is exposed to a strong, substantially constant static magnetic field. The static magnetic field causes the spin vectors of certain atomic nuclei within the body to randomly rotate or "precess" around an axis parallel to the direction of the static magnetic field. Radio frequency excitation energy is applied to the body, and this energy causes the nuclei to rotate or "precess" in phase and in an excited state. As the precessing atomic nuclei relax, weak radio frequency signals are emitted; such radio frequency signals are referred to herein as magnetic resonance signals.

Different tissues produce different signal characteristics. Relaxation times are the dominant factor in determining signal strength. In addition, tissues having a high density of certain nuclei will produce stronger signals than tissues with a low density of such nuclei. Relatively small gradients in the magnetic field are superimposed on the static magnetic field at various times during the process so that magnetic resonance signals from different portions of the patient's body differ in phase and/or frequency. If the process is repeated numerous times using different combinations of gradients, the signals from the various repetitions together provide enough information to form a map of signal characteristics versus location within the body. Such a map can be reconstructed by conventional techniques well known in the magnetic resonance imaging art, and can be displayed as a pictorial image of the tissues as known in the art.

The magnetic resonance imaging technique offers numerous advantages over other imaging techniques. MRI does not expose either the patient or medical personnel to X-rays and offers important safety advantages. Also, magnetic resonance imaging can obtain images of soft tissues and other features within the body which are not readily visualized using other imaging techniques. Accordingly, magnetic resonance imaging has been widely adopted in the medical and allied arts.

Many conventional magnetic resonance imaging instruments require that a patient lie on a horizontal bed that is then advanced into a tubular bore within a super-conducting solenoidal magnet used to generate the static magnetic field. Other conventional MRI imaging instruments use a magnet having a ferromagnetic frame defining a patient-receiving space. Considerable effort has been devoted to the design of such magnets in a manner that provides a relatively open patient-receiving space as opposed to the claustrophobic tubular bore of the conventional solenoidal magnet. However, in these instruments as well, the common practice is to provide the patient on a bed that remains horizontal throughout the procedure.

The position of a patient during magnetic resonance imaging may affect or limit the imaging information obtained. A patient may exhibit a symptom if oriented in an upright or weight bearing position and no symptom if oriented in a recumbent or horizontal position. For example, it may be necessary to image a patient in an upright or gravity bearing position to discern a symptom and provide a diagnosis for injuries relating to the neck, spine, hip, knee, foot or ankle areas of the human anatomy.

Advancement in magnetic resonance imaging has resulted in imaging apparatus that supports a patient in any position between a vertical position and a horizontal position. As described in greater detail in certain embodiments of commonly assigned United States Patents Nos. 6,414,490 and 6, 677, 753, a magnetic resonance imaging system can be provided with a patient support, such as a table, which can extend in a generally vertical direction so that the long axis of the patient is substantially vertical. For example, the patient may be in a standing posture, with his back, side or front leaning against a generally vertical patient support. Such a support may include a footrest projecting from the table at its lower end and the patient may stand on the footrest. In other arrangements, the support includes a seat projecting from the table so that the seat is in a horizontal plane when the table surface is vertical. In particularly preferred arrangements, the patient support can move relative to the magnet. For example, the patient support may be arranged to move vertically relative to the magnet so as to elevate a portion of the patient into the patient-receiving space of the magnet. Alternatively or additionally, the patient support may be arranged to tilt through a range of orientations between a generally horizontal orientation and a generally vertical orientation.

The magnets used in the preferred embodiments of the aforementioned '490 and '753 patents typically are arranged so that the magnetic field is directed along a horizontal axis, transverse to the axis of elongation of the patient support and hence transverse to the long axis of the patient. By contrast, in a conventional solenoidal magnet where the patient is received inside the bore of the solenoid, the magnetic field is directed along the long axis of the patient. U.S. Patent No. 5,349,956, shows a theoretical proposal for a conventional solenoidal magnet having its axis orientated in a vertical direction, with a patient elevator arranged to raise a standing patient into the bore. In theory, such an arrangement could provide for imaging of a patient in an upright position. In practice, this arrangement is never used. One aspect of the present invention incorporates the realization that there are mechanical and safety difficulties inherent in this approach, together with problems presented by a terrifying and claustrophobic environment for the patient.

European patent application 0 067 933 discloses a magnetic resonance imaging apparatus comprising a magnet having a bore extending along a vertical axis and capable of generating a magnetic field parallel to the vertical axis. The patient may sit on a seat during MR examination. Alternatively, the patient may be examined standing upright.

A similar apparatus is known from JP2001 104 276 where equipment is provided for transporting the patient into the bore of the magnet.

### SUMMARY OF THE INVENTION

The present invention concern a magnetic resonance imaging apparatus comprising a magnet having a bore extending along a vertical axis and capable of generating a magnetic field parallel to the vertical axis; and a patient support device and means arranged to translate said patient support device in a direction parallel to the vertical axis so that a portion of the patient support device becomes situated in said magnet bore, wherein the length of said magnet along a direction parallel to the vertical axis is 1.22 m (four feet) or less, and said magnet and patient support are rotatable between an upright position wherein said magnetic field is parallel to said vertical axis and a recumbent position transverse to said vertical axis when said portion of the patient support device is situated in said magnet bore.

In accordance with this aspect of the present invention, it is desirable that the apparatus also include a building structure housing said magnet and a magnet support structure connected to the building structure and connected to the magnet.

Further in accordance with this aspect of the present invention, the building structure includes a floor and the magnet support is connected to the floor.

Preferably, the magnet has an inner bore diameter of 91.4 cm (three feet) or more, however, an inner bore diameter of 45.7 cm (eighteen inches) or more is also suitable.

In a preferred embodiment in accordance with this aspect of the invention, the magnet comprises a solenoidal magnet having turns encircling the bore and adapted to generate a static magnetic field having a field direction coaxial with said bore.

In addition, it is desirable that the patient support be mounted to an elevator to raise and lower said patient support device if said magnet and patient support device are in the upright position.

It may also be preferable to provide a support structure having one end connected to a floor and another end connected to the magnet and wherein the patient support device is mounted on an elevator to raise and lower said patient support device if said magnet and patient support device are in the upright position.

Further in accordance with this aspect of the present invention, the magnet preferably has an inner bore diameter of 91.4 cm (three feet) or less.

In another embodiment the magnet and patient support are positionable in either the Trendelberg or reverse Trendelburg positions.

### BRIEF DESCRIPTION OF THE DRAWINGS

FIG. 1 is a perspective view of a magnetic resonance imaging apparatus in accordance with an aspect of the present invention.
FIG. 2A is a perspective view of a magnet resonance imaging apparatus in accordance with an aspect of the present invention.
FIG. 2B is a front view of the magnet apparatus of FIG. 2A.
FIG. 2C is a partial exploded view of a portion of the magnet apparatus of FIG. 2A.
FIG. 2D illustratively depicts an embodiment of a portion of a rod member which can be used with the present invention.
FIG. 3 is a perspective view of a short bore magnet which can be used in an apparatus in an apparatus of the present invention.
FIG. 4 is a perspective view of a magnetic resonance imaging apparatus in accordance with an aspect of the present invention.
FIG. 5 is a perspective view of a magnetic imaging apparatus in accordance with an aspect of the present invention.
FIG. 6 is an illustrative schematic of a facility in accordance with an aspect of the present invention.

### BEST MODE FOR CARRYING OUT INVENTION

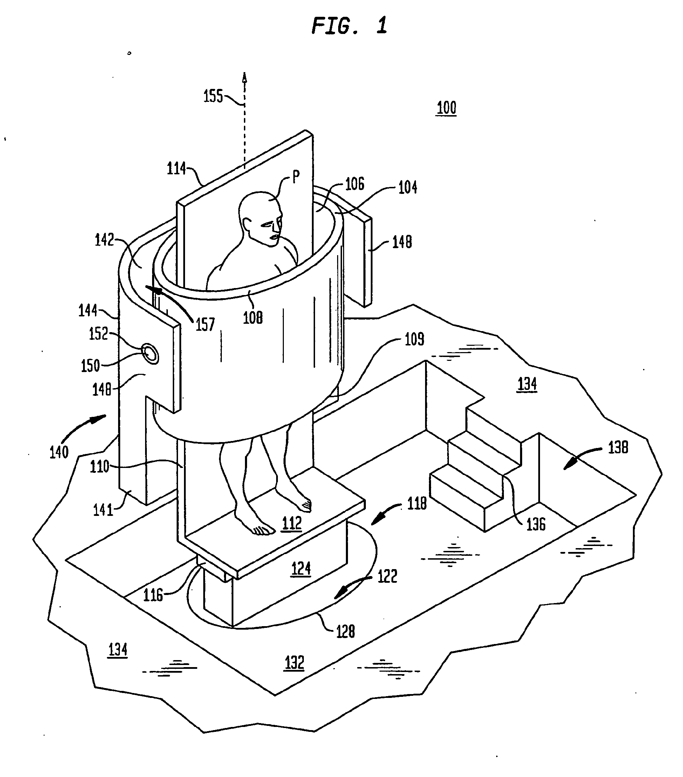

FIG. 1 is a perspective view of a magnetic resonance imaging apparatus 100 in accordance with an aspect of the present invention. The apparatus 100 includes a magnet 104 having a substantially cylindrical central bore 106. The magnet 104 is preferably an electromagnet and most preferably a superconducting solenoidal magnet. A patient support device or table 110 is positionable within the bore 106 and, as shown, may protrude through each end 108, 109 of the magnet 104. As illustrated, the patient support device 110 is capable of supporting a patient P in a standing position with the patient's head and feet being positioned outside the bore. The patient support device 110 may also be constructed so as to support a patient in a seated or sitting position. The patient support device 110 includes a footrest 112 at one end, which provides a standing surface for a patient. The patient support includes a distal or free end 114 opposite the footrest 112. When a patient is oriented in a standing position, the patient's head is located towards the free end 114. The patient support may also include a seat (not shown) that allows a patient to sit while imaged. The patient support device may be made in accordance with the patient support device disclosed in pending U.S. Patent Application Publication 2003/0204136 A1. filed on April 25, 2002; 2004/0030241 A1 filed on may 1, 2003 which are assigned to assignee of the present invention.

As further illustrated in FIG. 1, the footrest 112 of the patient support device 110 is preferably attached or mounted on a pedestal 116 of an elevator 118. The elevator 118 is disposed within a well 122. The elevator 118 includes a shaft member 124. The shaft member 124 may comprise a plurality of co-centric cylindrical shaped members that recess into one another and an opening 128, e.g., telescoping. Alternatively, the shaft member may comprise a plurality of rectangular or square shaped members that recess into one another and into the opening 128. Although other arrangements for an elevator may be used, the use of shaft members wherein the rectangular or cylindrical shaped members recess into each other aids in minimizing the vertical space required beneath the well floor 132. Preferably, the opening 128 for the shaft member 124 extends approximately 61 cm (two feet) beneath the floor 132 of the well 122. Access to the patient support device 110 is provided by a series of steps 134 to an open area, well or pit 138. In a preferred embodiment, the floor 132 of the area 138 is at a depth of approximately 91.4 cm (3 feet) beneath the floor 134.

The apparatus also includes a support structure 140 for the magnet 104. As shown, the support structure 140 includes one end 141 proximate the floor 134. The support structure 140 also includes an inner sidewall 142 and an outer sidewall 144. The inner and outer sidewalls 142, 144 are curved to accept the shape of the magnet. The inner and outer sidewalls, 142, 144 terminate on L-shaped end walls 148, 148, which are arranged substantially opposite each other. Most preferably, each end wall 148 includes an opening through which is inserted a rod member 150. As shown, an end of the rod member 150 includes a flange 152 the overlaps the end wall 148 opening so as to keep the rod member 150 in place. The other end (not shown) of the rod member 150 is connected to the magnet 104. The arrangement of the rod member 150 allows the magnet 104 to tilt relative to a vertical axis 155. Accordingly, the apparatus preferably includes a space 157 between the inner sidewall 142 and the rearward outer wall of the magnet 104.

In addition to acting as a support for the magnet 104, the support structure 140 also serves as a housing through which the power supply and cooling flow necessary to operate a magnet may be fed to the magnet 104. Accordingly, the inner sidewall 142 may also include an opening through which cables and other conduits may be attached to the magnet 104. Where the magnet 104 is a superconducting magnet, a main magnet coil and a correction magnet coil may be wound within the interior of magnet structure 104. A cryocooler or other suitable cooling agent passes through conduits in the support structure 140 to cool both the main and correction magnet coils. In addition, suitable electrical connections may also be provided via the support structure to energize both the main coil and the correction coil and to provide a signal for controlling the tilt of the magnet 104.

The distance between the lower end 109 and the floor 134 is chosen so as to allow enough clearance space between the free end 114 of the patient support device 110 and the lower end 109 of the magnet 104 so that a patient can step onto the foot rest 112 when the elevator 118 is at its lowest point without hitting their head against the lower end 109. Thus, preferably the distance between the well floor 132 and the lower end 109 is at least 183 cm (six feet). Most preferably, the lower end 109 of the magnet 104 is suspended approximately 91.4 cm (three feet) above the floor 134 and 183 cm (six feet) above the well floor 132. In such an arrangement, the length of the patient support 110, as measured between the footrest 112 and the free end 114, is slightly less than 183 cm (six feet) so that the patient support sits entirely beneath the lower end 109 of the magnet when the elevator 118 is completely recessed within the opening 128.

The distance between the lower end 109 of the magnet and the floor 134 or the distance between the floor 132 and the floor 134 (i.e., the well or pit depth) may be suitably arranged to allow for more room between the lower end 109 of the magnet and the free end 114 of the patient support. For example, the lower end 109 of the magnet may be positioned approximately 122 cm (four feet) above the floor 134 with a bit depth of three feet. Alternatively, the lower end 109 of the magnet may be positioned approximately 91.4 cm (three feet) above the floor 134 with a "pit" depth of 122 cm (four feet).

In accordance with another aspect of the present invention, a patient can be imaged as follows. Initially, the elevator 118 is adjusted to its lowest position such that the planar surface of the footrest 112 is substantially parallel with the well floor 132. A patient P enters the well or pit area 138 using steps 136 and proceeds to stand on the footrest 112. The elevator 118 is then raised and the patient enters the bore 106 headfirst. An operator then adjusts the height of the elevator 118 such that that portion of the patient's anatomy to be imaged will be appropriately positioned within the imaging volume of the magnet 104. Preferably, the coils of the magnet would have been previously energized (e.g., at the start of the day) to create a static magnetic field that is oriented substantially vertically and in a direction substantially parallel to the vertical axis 155, which is substantially parallel to the long axis of the patient. Imaging then proceeds as is known in the art. Once imaging is completed the elevator is lowered so that the planar surface of the footrest 112 is again substantially parallel with the well floor 132. The patient may then step off footrest 112 and exit the pit or well area 138.

As previously discussed, the magnet 104 may include a mechanism that allows the magnet to tilt relative to the vertical axis 155. Thus, in some instances the imaging process may also include tilting the magnet and acquiring images of the patient with the magnet at an angle relative the patient.

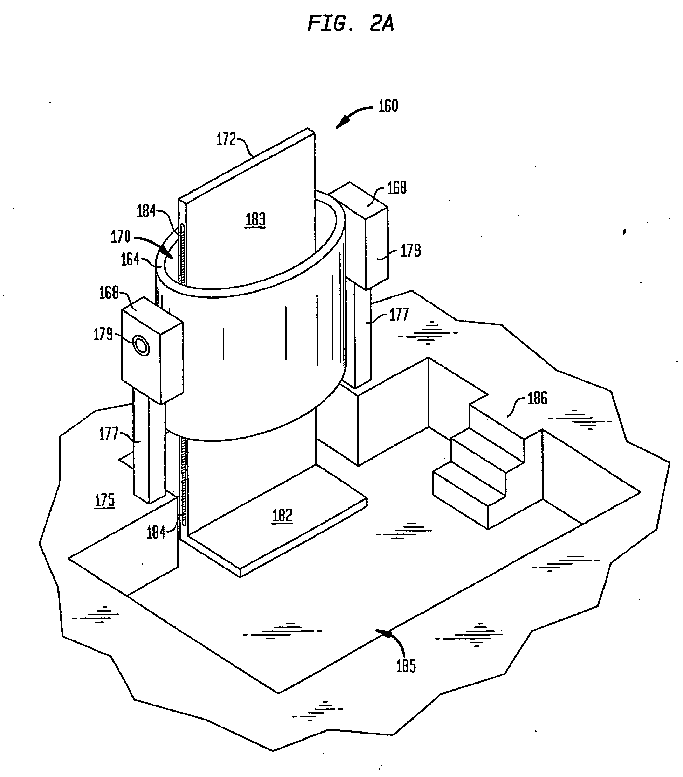

FIG. 2A illustratively depicts a perspective view of a short bore magnet apparatus 160 in accordance with an aspect of the present invention. The apparatus 160 includes a short bore magnet 164 and a support structure 168. The magnet 164 is most preferably a superconducting solenoidal magnet and includes a bore 170. A patient support device 172 is supported by support structures 168, 168 and positionable within the bore 170. In addition to providing support to the magnet 164 and device 172, the support structure 178 also provides a housing and conduits for supporting power and cooling necessary to operate the magnet 164. Thus, wiring and piping may enter the support structure through the floor 175 at legs 177, 177 and be fed to the magnet 164 through the box 179 of the housing 168.

The device 172 includes a footrest 182 for supporting the feet of a standing patient and a support surface 183 against which either the anterior or posterior surface of a patient's anatomy may be supported. The device 172 also includes a pair of slots 184 along its longitudinal edges 186. The slots 184 are used to translate the device into and out of the bore 170 of the magnet 164. As seen in FIG. 2A, the device 172 is disposed above a well 185, which may be entered by steps 186.

In operation, a patient enters the well 185 via steps 186 while the device 172 is lowered to the floor 187 of the well 185. Once the patient is positioned on the footrest 182, the device 172 is raised, via gears housed in box 179, so that that portion of the patient's anatomy of interest may be imaged.

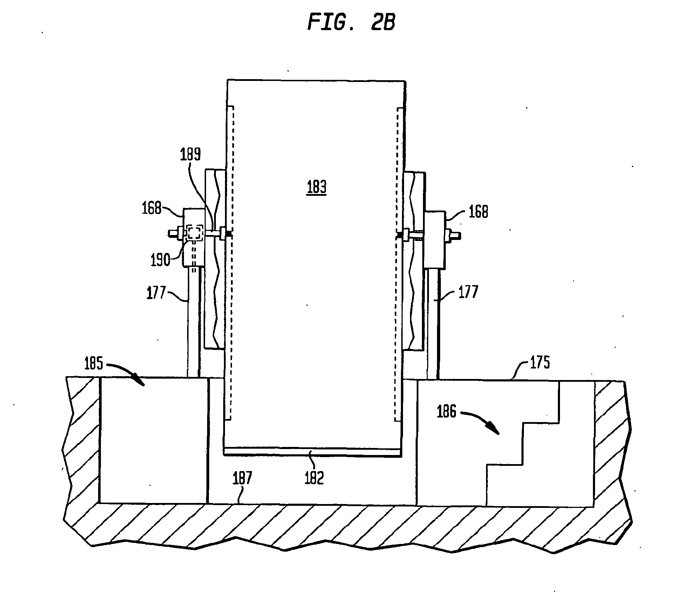

In addition to allowing for imaging in an upright position, the apparatus 160 allows the patient to be also imaged in any position between horizontal and vertical, and the Trendelenburg and reverse Trendelenburg positions by rotating the magnet 164 and device 172. As best seen in FIG. 2B, the magnet 164 and device 172 are rotatably mounted to the support structure 168. In a preferred embodiment, a rod member 189 is mounted to gears 190, the device 172 and the magnet 174.

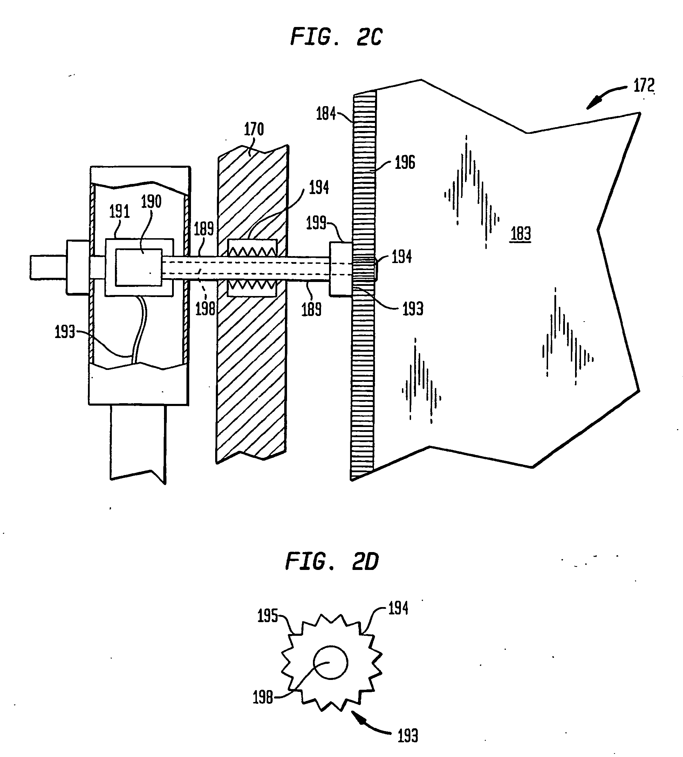

As best seen in FIG. 2C, the rod member 189 and gear 190 are coupled together in block 191 and are preferably electrically controlled and operated via cables 192, which extend downward to the floor. In the magnet 170, the rod member 189 is mounted into a sleeve 194 that rotates in unison with the rod 189. The rod member 189 terminates on the longitudinal edge of the device 172 in the slots 184. As shown, the end portion 193 of the rod member 189 disposed within slot 184 may include a larger cylindrical diameter than the rest of the rod, so that grooves 194 formed on the end portion 193 may reliably support, rotate and translate the device 172.

As best seen in FIG. 2D, the grooves 194 form pawls or fingers 195 that engage slots 196 on the device 172. In the embodiment shown, translation of the device 172 relative to the bore 170 of the magnet 168 occurs when inner rod 198 (see FIG. 2D), which may form a unitary structure with end portion 193, rotates within the rod member 189. Thus, where rotation of the magnet 168 and device 172 is desired, the inner rod 198 remains fixed relative to rod member 189 and the device 172 rotates via rod member 189 at end cap 199.

Although in the embodiment shown in FIG. 2, the rotation of the magnet and device and translation of device are discussed in relation to one of the support structure 168, the same or similar rotational and translation means may be incorporated on the other support structure 168. Thus, in a variant the mechanism for rotation and translation may be implemented on either support structure or both support structures. In some instances it may be preferable to have the rotational and translation means included only in a single support structure, while using the other support to provide any other ancillary functions, e.g., cable and conduit routing, necessary to operate the apparatus. In addition to the particular rotation and translation disclosures discussed above, other structures known in the art for similar functionality may be provided accordingly.

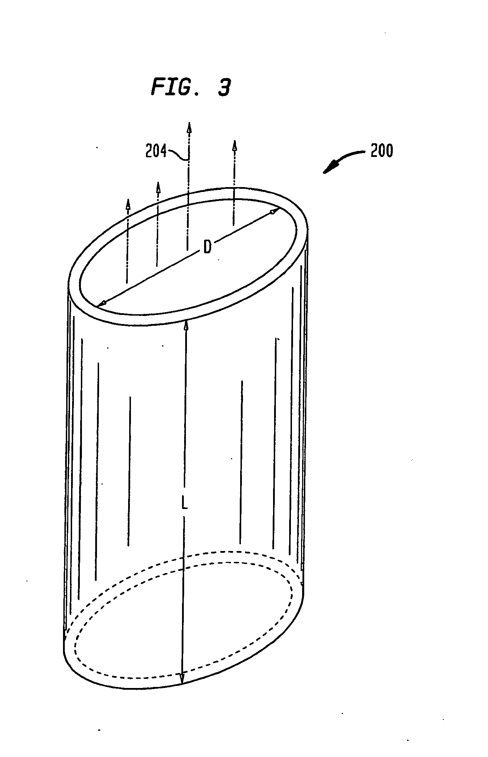

As best seen in FIG. 3, in a preferred embodiment a magnet 200 in accordance with the present invention includes a central cylindrical bore having a diameter, D, of approximately 45.7 cm (eighteen inches) or more. In addition, the length of the magnet, L, as measured along a cylindrical axis 204 is preferably approximately 91.4 cm (three feet). The diameter D of the magnet impacts the field strength and homogeneity of the static magnetic field that is produced when the magnet coils are energized. Thus, as the internal diameter D is increased, the strength and homogeneity of the magnetic field within the bore may be affected. However, the diameter D of the magnet 200 may be as large as 91.4 cm (three feet) or more without sacrificing too much in the way of image acquisition by increasing the number of ampere-turns if necessary.

As discussed above, the well depth and distance between the lower end 109 of the magnet and floor 134 is advantageously chosen to provide a comfortable clearance space between the lower end 109 of the magnet as the patient steps onto and off the foot rest 112. Thus, in a preferred embodiment well 122 is approximately 91.4 cm (three feet) in depth and the magnet 104 is positioned such that its lower end 109 is approximately 91.4 cm (three feet) above the floor. With a magnet having a length, L, of three feet, an apparatus, in accordance with the present invention, may require a floor 134 to ceiling clearance of approximately 2.74 cm (nine feet) for full body scanning. The floor 134 to ceiling clearance space requirement may be suitably lowered by increasing the well depth so that the apparatus may be housed in facilities that include less than nine feet of ceiling clearance space.

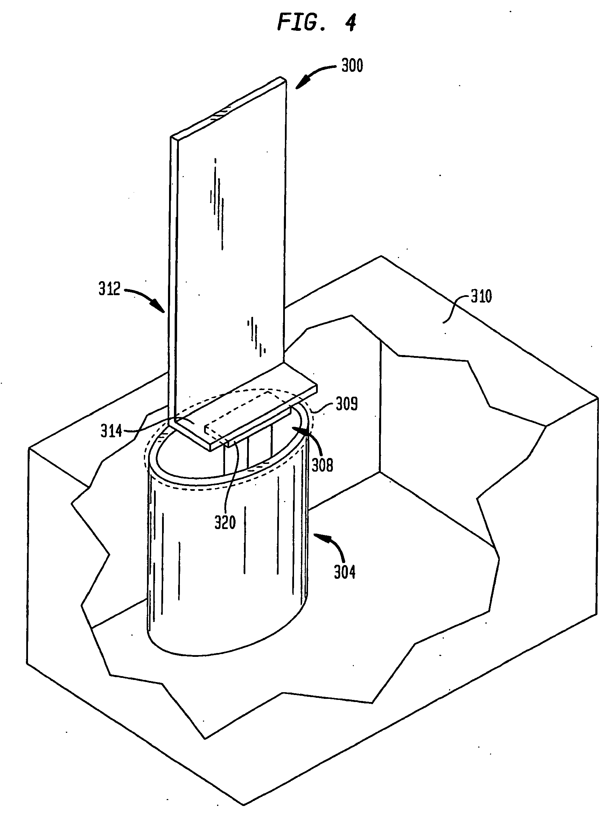

FIG. 4 illustrates a magnetic resonance imaging apparatus 300 in accordance with an aspect of the present invention. The apparatus includes a magnet 304 preferably having a substantially cylindrical center bore 308, which is disposed in an opening 309 beneath a floor 310. The magnet 304 may be of the type discussed above in reference to FIGS. 1, 2 and 3. A patient support member 312 having a base member 314 and a wall 316 substantially transverse to the base member 314 is disposed in the bore 308. The patient support member 312 may be used as standing area for a patient. The patient support member 312 is mounted on an elevator 320. The elevator is used to lower and raise the patient support member 312 within the bore 308. As discussed, the apparatus 300 is preferably disposed beneath the floor 310. Preferably, the opening 310 for the magnet is contoured such that it completely hides the magnet 304. In accordance with an aspect of the present invention, a patient may sit on the floor 310 with his or her leg hanging in the bore so that the patient's lower extremities may be imaged. In accordance with this aspect of the present invention, the apparatus 300 may be used to image various portions of the lower extremities of a human anatomy, e.g., legs, ankle, knees, with the patient in both a standing and sitting position.

In addition to being configurable to image the lower extremities of a patient, the apparatus 300 may also be configured to image any portion of the human anatomy by allowing the elevator to recess further below the floor 310 into the magnet bore 308. Thus, the apparatus 300 may be used to image the torso of the anatomy. Although the head may also be imaged, in order to avoid creating feelings of anxiety and claustrophobia, it may be preferable to limit imaging in apparatus 300 to below the neck of a patient. Nonetheless, under the appropriate circumstances, the entire body, including the head of a patient, may be imaged using apparatus 300.

It may also be advantageous, from a patient's perspective, to house the apparatus 300 beneath the floor such that it is concealed from the patient. In such an embodiment, auxiliary lighting may be employed to illuminate the magnet's bore space to make the scanning experience more comfortable.

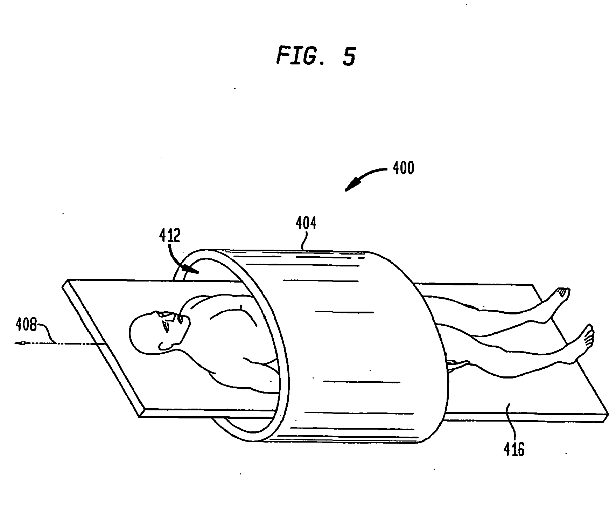

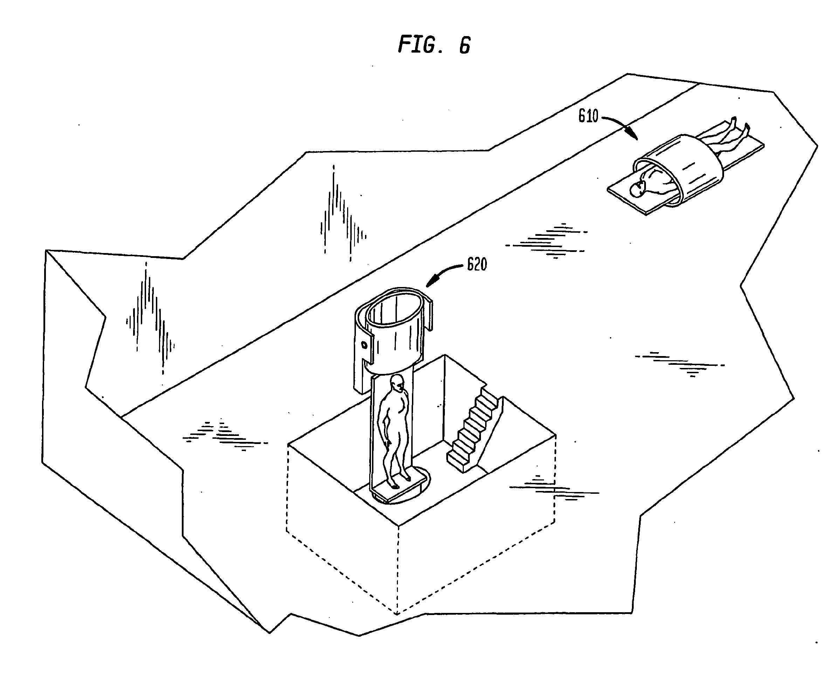

Another aspect of the present invention is a facility for performing magnetic resonance imaging. In accordance with this aspect of the invention, the facility is equipped with a short bore vertical field magnet as described hereinabove and a short bore horizontal field magnet. In accordance with this aspect of the present invention, a short bore horizontal field magnet may be constructed as illustrated in FIG. 5. The apparatus 400 comprises a short bore magnet 404 having a horizontal field axis 408 that extends along a substantially horizontal direction. The magnet 404 includes a bore 412 into which may be placed a patient support 416. The magnet 404 may be of dimensions similar to those discussed hereinabove in relation to FIGS. 1, 2 and 3. Although not shown, the magnet 404 may include various support structures, similar to those disclosed in FIG. 1, to hold the magnet in place. In addition, various arrangements are available for translating the patient support 416 parallel to the horizontal field axis 408. A facility equipped with both horizontal and vertical field short bores may advantageously increase the throughput of the facility. In particular, and as shown in FIG. 6, a facility that includes both a short bore horizontal field magnet 610 and a vertical field magnet 620 may advantageously increase the imaging throughout capability by allowing patients to be imaged in either apparatus. Advantageously, the magnet 620 would be designated for imaging where having the weight bearing position may yield more meaningful information. Alternatively, where it is acceptable that imaging be performed in a horizontal position, magnet 610 may be used. Furthermore, although the magnet 620 is shown as being positioned above the floor as in FIGS. 1 or 3, the apparatus of FIG. 4 may comprise the vertical field magnet.

A variant in accordance with the present invention may include a magnet assembly comprising a plurality of coils arranged asymmetrical along an axis such that the center of a static magnetic field generated by the coils is offset from the center of the assembly as described, for example, in U.S. Patent No. 4,689,591 to McDougall. In accordance with this aspect of the present invention, the coils are oriented to provide a static magnetic field having a field axis oriented in a substantially vertical direction as described above. A patient support device may then be appropriately equipped so as to raise or lower a patient into and out of the static magnetic field for imaging.

A variant in accordance with the present invention may include replacement of either of the magnets 104, 160, 200, 304 or 400 with a pancake magnet of the type disclosed in U.S. Patent No. 5,428,292 to Dorri et al. ("the '292 patent"). Further in accordance with this aspect of the present invention, one or more of the '292 patent magnets may be stacked apart to provide a more open feeling to a patient being imaged. Thus, the magnets may be stacked vertically using assembly posts to provide an open magnetic resonance imaging system. The stacked magnets would then mounted to a support arm or structure and a patient could then be imaged in the bore created by the stacked magnets. In another variant in accordance with the present invention the magnets 104, 160, 200, 304 or 400 may be replaced with magnet assemblies described in accordance with U.S. Patent Nos. 5,291,169 to Ige et al. ("the '169 patent") or 6,078,234 to Huang, et al. of "the '234 patent"). These magnet assemblies desirably comprise a separated pair of annular superconducting magnet assemblies in an open architecture. In accordance with an aspect of the present invention, the magnets of the '169 or '234 patents may be turned on its end to provide a static magnetic field directed substantially in a vertical direction, thereby allowing a standing patient to be imaged within the bore of the magnet assembly.

Although the invention herein has been described with reference to particular embodiments, it is to be understood that these embodiments are merely illustrative of the principles and applications of the present invention. For example, other magnet assemblies such as that described in U.S. Patent No. 4,721,914 to Fukushima et al. may be applied or used in accordance with the present invention. It is therefore to be understood that numerous modifications may be made to the illustrative embodiments and that other arrangements may be devised without departing from the scope of the present invention as defined by the appended claims.

## Claims

1. A magnetic resonance imaging apparatus (100, 160, 300, 400), comprising:
a magnet (104, 164, 200, 304, 404) having a bore extending along a vertical axis and capable of generating a magnetic field parallel to the vertical axis; and
a patient support device (110, 172, 416) and means arranged to translate said patient support device in a direction parallel to the vertical axis so that a portion of the patient support device becomes situated in said magnet bore,
wherein the length of said magnet (104, 164, 200, 304, 404) along a direction parallel to the vertical axis is 1.22 m (four feet) or less, and
said magnet and patient support are rotatable between an upright position wherein said magnetic field is parallel to said vertical axis and a recumbent position transverse to said vertical axis, when said portion of the patient support device is situated in said magnet bove.

2. The apparatus of claim 1, further comprising a building structure housing said magnet (104, 164, 200, 304, 404) and a magnet support structure (140, 168, 178) connected to said building structure and said magnet.

3. The apparatus of claim 2, wherein said building structure includes a floor (134) and said magnet support is connected to said floor.

4. The apparatus of claim 1, wherein said magnet (104, 164, 200, 304, 404) has an inner bore diameter of 45.7 cm (eighteen inches) or more.

5. The apparatus of claim 4, wherein said magnet (104, 164, 200, 304, 404) has an inner bore diameter of 91.4 cm (three feet) or more.

6. The apparatus of claim 1, wherein said magnet (104, 164, 200, 304, 404) comprises a solenoidal magnet having turns encircling said bore and adapted to generate a static magnetic field having a field direction coaxial with said bore.

7. The apparatus of claim 1, further comprising an elevator (320) on which said patient support device is mounted if said magnet and patient support are in the upright position, said elevator being arranged to raise and lower said patient support device.

8. The apparatus of claim 1, further comprising a support structure having one end to be supported by a floor and another end connected to said magnet, the apparatus comprising elevator on which said patient support device is mounted if said magnet and patient support are in the upright position.

9. The apparatus of claim 1, wherein the length of said magnet along a direction parallel to the vertical axis is less than 91.4 cm (three feet).

10. The apparatus of claim 1, wherein said magnet and patient support are positionable in a Trendelenburg position.

11. The apparatus of claim 10, wherein said magnet and patient support are positionable in a reverse Trendelenburg position.

## Patentansprüche

1. Magnetresonanz-Bildgabevorrichtung (100, 160, 300, 400), umfassend:
einen Magneten (104, 164, 200, 304, 404) mit einer Bohrung, die sich entlang einer vertikalen Achse erstreckt und in der Lage ist, ein Magnetfeld parallel zu der vertikalen Achse zu erzeugen; und
eine Patientenauflagevorrichtung (110, 172, 416) und ein Mittel, das dafür konfiguriert ist, die Patientenauflagevorrichtung in einer Richtung parallel zu der vertikalen Achse so zu verschieben, dass sich ein Abschnitt der Patientenauflagevorrichtung in die Bohrung des Magneten hineinbewegt,
wobei die Länge des Magneten (104, 164, 200, 304, 404) entlang einer Richtung parallel zu der vertikalen Achse 1,22 m (vier Fuß)oder weniger beträgt, und
wobei der Magnet und die Patientenauflage zwischen einer aufrechten Position, in der das Magnetfeld parallel zu der vertikalen Achse verläuft, und einer liegenden Position quer zu der vertikalen Achse, wenn sich der Abschnitt der Patientenauflagevorrichtung in der Bohrung des Magneten befindet, drehbar sind.

2. Vorrichtung nach Anspruch 1, die des Weiteren eine Gebäudestruktur, die den Magneten (104, 164, 200, 304, 404) aufnimmt, und eine Magnetstützkonstruktion (140, 168, 178), die mit der Gebäudestruktur und dem Magneten verbunden ist, umfasst.

3. Vorrichtung nach Anspruch 2, wobei die Gebäudestruktur einen Fußboden (134) enthält und die Magnetstützkonstruktion mit dem Fußboden verbunden ist.

4. Vorrichtung nach Anspruch 1, wobei der Magnet (104, 164, 200, 304, 404) einen Bohrungsinnendurchmesser von 45,7 cm (achtzehn Zoll) oder mehr aufweist.

5. Vorrichtung nach Anspruch 4, wobei der Magnet (104, 164, 200, 304, 404) einen Bohrungsinnendurchmesser von 91,4 cm (drei Fuß) oder mehr aufweist.

6. Vorrichtung nach Anspruch 1, wobei der Magnet (104, 164, 200, 304, 404) einen Solenoid-Magneten umfasst, der Windungen aufweist, welche die Bohrung umkreisen, und dafür geeignet ist, ein statisches Magnetfeld zu erzeugen, dessen Feldrichtung koaxial zu der Bohrung verläuft.

7. Vorrichtung nach Anspruch 1, des Weiteren einen Aufzug (320) umfassend, auf dem die Patientenauflagevorrichtung montiert ist, wenn sich der Magnet und die Patientenauflage in der aufrechten Position befinden, wobei der Aufzug dafür konfiguriert ist, die Patientenauflagevorrichtung anzuheben und abzusenken.

8. Vorrichtung nach Anspruch 1, die des Weiteren eine Stützstruktur umfasst, die ein Ende aufweist, das durch einen Fußboden zu stützen ist, und ein weiteres Ende aufweist, das mit dem Magneten verbunden ist, wobei die Vorrichtung einen Aufzug umfasst, auf dem die Patientenauflagevorrichtung montiert ist, wenn sich der Magnet und die Patientenauflage in der aufrechten Position befinden.

9. Vorrichtung nach Anspruch 1, wobei die Länge des Magneten entlang einer Richtung parallel zu der vertikalen Achse kleiner als 91,4 cm (drei Fuß) beträgt.

10. Vorrichtung nach Anspruch 1, wobei der Magnet und die Patientenauflage in einer Trendelenburg-Position positioniert werden können.

11. Vorrichtung nach Anspruch 10, wobei der Magnet und die Patientenauflage in einer umgekehrten Trendelenburg-Position positioniert werden können.

## Revendications

1. Appareil d'imagerie à résonance magnétique (100, 160 300, 400), comprenant:
un aimant (104, 164, 200, 304, 404) ayant un alésage s'étendant le long d'un axe vertical et capable de générer un champ magnétique parallèle à l'axe vertical; et
un dispositif de support de patient (110, 172, 416) et un moyen de translation dudit dispositif de support de patient dans une direction parallèle à l'axe vertical de sorte qu'une portion du dispositif de support de patient devienne située dans ledit alésage d'aimant, dans lequel la longueur dudit aimant (104, 164, 200, 304, 404) dans une direction parallèle à l'axe vertical est de 1,22 m (quatre pieds) ou moins, et
ledit aimant et ledit support de patient peuvent être tournés entre une position debout à laquelle ledit champ magnétique est parallèle au dit axe vertical et une position couchée transversale au dit axe vertical lorsque ladite portion du dispositif de support de patient est située dans ledit alésage d'aimant.

2. Appareil selon la revendication 1, comprenant en outre une structure de construction hébergeant ledit aimant (104, 164, 200, 304, 404) et une structure de support d'aimant (140, 168, 178) reliée à ladite structure de construction et au dit aimant.

3. Appareil selon la revendication 2, dans lequel ladite structure de construction comprend un plancher (134) et ledit support d'aimant est relié au dit plancher.

4. Appareil selon la revendication 1, dans lequel ledit aimant (104, 164, 200, 304, 404) a un diamètre d'alésage intérieur de 45,7 cm (huit pouces) ou plus.

5. Appareil selon la revendication 4, dans lequel ledit aimant (104, 164, 200, 304, 404) a un diamètre d'alésage intérieur de 91,4 cm (trois pieds) ou plus.

6. Appareil selon la revendication 1, dans lequel ledit aimant (104, 164, 200, 304, 404) comprend un aimant solénoïdal comportant des tours encerclant ledit alésage et apte à générer un champ magnétique statique ayant une direction de champ coaxiale au dit alésage.

7. Appareil selon la revendication 1, comprenant en outre un élévateur (320) sur lequel ledit dispositif de support de patient est monté si ledit aimant et ledit dispositif de support de patient se trouvent à la position debout, ledit élévateur étant agencé pour soulever et abaisser ledit dispositif de support de patient.

8. Appareil selon la revendication 1, comprenant en outre une structure de support ayant une extrémité à supporter par un plancher et une autre extrémité reliée au dit aimant, l'appareil comprenant un élévateur sur lequel ledit dispositif de support de patient est monté si ledit aimant et ledit dispositif de support de patient se trouvent à la position debout.

9. Appareil selon la revendication 1, dans lequel la longueur dudit aimant dans une direction parallèle à l'axe vertical est inférieure à 91,4 cm (trois pieds).

10. Appareil selon la revendication 1, dans lequel ledit aimant et ledit dispositif de support de patient peuvent être positionnés à une position de Trendelenburg.

11. Appareil selon la revendication 10, dans lequel ledit aimant et ledit dispositif de support de patient peuvent être positionnés à une position de Trendelenburg inversée.
